(19) **Europäisches Patentamt**
**European Patent Office**
**Office européen des brevets**

(11) **EP 2 626 701 B1**

(12) **EUROPEAN PATENT SPECIFICATION**

(45) Date of publication and mention of the grant of the patent:
**25.03.2015 Bulletin 2015/13**

(51) Int Cl.:
***G01N 33/50*** *(2006.01)*

(21) Application number: **13154536.0**

(22) Date of filing: **08.02.2013**

(54) **Method for detecting a xenoantigen in fixed tissues used as bioprosthetic replacements**

Verfahren zur Erkennung eines als Bioprothesenersatz verwendeten Xenoantigens in fixiertem Gewebe

Procédé pour détecter un xénoantigène dans des tissus fixés utilisés comme substituts bioprothétiques

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.02.2012 IT PD20120029**

(43) Date of publication of application:
**14.08.2013 Bulletin 2013/33**

(73) Proprietor: **Anthropos Research S.r.l.**
**45031 Arquà Polesine (RO) (IT)**

(72) Inventors:
• **NASO, Filippo**
**35045 Ospedaletto Euganeo (IT)**
• **GANDAGLIA, Alessandro**
**35030 Rubano (IT)**
• **SPINA, Michele**
**30123 Venezia (IT)**
• **GEROSA, Gino**
**37042 Caldiero (IT)**

(74) Representative: **Gervasi, Gemma et al**
**Notarbartolo & Gervasi S.p.A.**
**Corso di Porta Vittoria 9**
**20122 Milano (IT)**

(56) References cited:
**EP-A2- 0 795 337**

• **K. Z. KONAKCI ET AL: "Alpha-Gal on bioprostheses: xenograft immune response in cardiac surgery", EUROPEAN JOURNAL OF CLINICAL INVESTIGATION, vol. 35, no. 1, 1 January 2005 (2005-01-01), pages 17-23, XP055029213, ISSN: 0014-2972, DOI: 10.1111/j. 1365-2362.2005.01441.x**
• **MCGREGOR C G A ET AL: "Cardiac xenotransplantation technology provides materials for improved bioprosthetic heart valves", JOURNAL OF THORACIC AND CARDIOVASCULAR SURGERY, MOSBY-YEAR BOOK, INC., ST. LOUIS, MO, US, vol. 141, no. 1, 1 January 2011 (2011-01-01), pages 269-275, XP027559864, ISSN: 0022-5223 [retrieved on 2010-12-16]**
• **Dan T Simionescu: "Artificial Heart Valves", Wiley Encyclopedia of Biomedical Engineering, 14 April 2006 (2006-04-14), pages 1-10, XP055029091, Retrieved from the Internet: URL: http://onlinelibrary.wiley.com/store/1 0.1002/9780471740360.ebs1455/asset/ebs1455 . pdf?v=1&t=h348ck79&s=f38817f11fb5a52211c6 62e4015dc1dbe3073870 [retrieved on 2012-06-06]**
• **F. NASO ET AL: "First quantitative assay of alpha-Gal in soft tissues: Presence and distribution of the epitope before and after cell removal from xenogeneic heart valves", ACTA BIOMATERIALIA, vol. 7, no. 4, 1 April 2011 (2011-04-01), pages 1728-1734, XP055029215, ISSN: 1742-7061, DOI: 10.1016/j.actbio. 2010.11.030**

**EP 2 626 701 B1**

**(Cont. next page)**

• Peter R Andreana ET AL: "Alpha-Galactosyl trisaccharide epitope: Modification of the 6-primary positions and recognition by human anti-[alpha]Gal antibody", Glycoconjugate Journal, 1 January 2004 (2004-01-01), pages 107-118, XP055029184, Retrieved from the Internet: URL:http://www.springerlink.com/content/p5 291nh9t7871n2x/fulltext.pdf [retrieved on 2012-06-07]

**Description**

**Field of the invention**

The invention relates to a method for detection of a xenoantigen, in particular the α-Gal epitope, in fixed xenogeneic tissues, in particular glutaraldehyde-fixed tissues, which can be employed to manufacture bioprosthetic replacements for use in the human clinical field.

**Background of the invention**

Recent advances in tissue engineering of the bioprosteses provided a substantial boost of this sector, either commercially or clinically, to investigate new scenarios in order to contribute in the worldwide improvement of healthcare cures.

In such sense the cardiovascular area of interest should be considered symbolic both in terms of the social and healthcare impact and of the established procedure for the replacement of heart valves.

Indeed, heart valves dysfunction have a high impact on population worldwide as there are various aetiological causes. Modifications of the valve apparatus induced by inflammatory *phenomena* (such as rheumatic diseases), infectious (such as endocarditis) or degenerative conditions (myxoid degeneration, calcific dystrophy) may induce functional and morphological alteration (stenosis or insufficiency) with severe impairment of blood circulation, often incompatible with life itself. The impact of these valvulopathies on public health is also linked to the correlation between the degenerative aetiology and the progressive ageing of the population. The most recent epidemiological surveys carried out in Europe and in USA [1] show a prevalence of moderate-severe aortic stenosis in 4.6 % of the population aged >75 years of age, which reaches 8.1 % after 85 years of age. From the moment it becomes symptomatic or determines left ventricular dysfunction, aortic stenosis is a disease with poor prognosis in the short term.

The discovery of the "heart-lung machine" in the 1960s allowed cardiac function to be temporarily blocked through the use of extracorporeal circulation, thus permitting surgical operations on a motionless heart muscle. Biomedical technology has, therefore, became capable of developing and surgically applying for valve prosthesis substitutes suitable to imitating the natural function of native and not pathological valves. At present, valve replacement is the elective therapy eligible to ameliorate patients' prognosis and significantly improving their quality of life.

The ideal valve prosthesis should allow a transvalvular flow comparable to that of the corresponding native healthy valve, with a long lifespan and not generating haemolytic or thrombogenic effects. Since these characteristics are generally difficult to obtain when condensed in a single device, the current commercial cardiac valve prostheses result from consistent modifications and constant improvements that are still on-going. Such prostheses, available for clinical purposes, are divided into mechanical, biological and homograft prostheses. Mechanical prostheses do not generally encounter any degeneration over time, however they require anticoagulant therapy. They cannot be repopulated by cells, as they do not provide a biological support that promotes cell attachment. This compromises the possibility of a structural remodelling and thus for example, an increase in size in relation to the patient's body growth. They can last for up to about 20 years, beyond which they must be replaced due to the formation of fibrous *panniculus,* not compatible with the leaflets motility.

On the other hand, homograft means a non-xenogeneic valve prostheses collected during autopsies from hearts unsuitable for entire organ transplantation, belonging to individuals aged between 18 and 65. They include a portion of about 5-6 cm of ascending aorta or pulmonary artery and the fibrous annulus of the valve itself. The advantages of these bioprostheses, once implanted, are due to their excellent hemodynamic profile and low thromboembolic and infection risk. Moreover, homografts do not require anticoagulation therapy. Nevertheless, these types of bioprostheses have limited time duration: homografts degeneration results from calcific dystrophy phenomena in a manner that is inversely proportional with the age of the recipient (this aspect is due to an increased physiological mobilization of calcium, that in paediatric and young patients is associated with the development of the skeletal apparatus). In addition, the high number of hospitalizations, requiring valve replacement surgery, and the low availability of such biological prostheses (obtained from deceased donors), makes them insufficient in number to meet clinical demand.

Because of these drawbacks, the most commonly valves used as replacement are represented by biological prostheses deriving from xenogeneic tissues, in particular deriving from pig valves or from bovine or equine pericardium. These bioprostheses are produced either with a semi-rigid mean supporting the fibrous annulet and used as an anchor in surgical procedures (stented bioprostheses) or without such mean of support (stentless bioprostheses). The blood flow throughout these bioprostheses is physiologically similar to that of native valves and the therapy after valve replacement does not require anticoagulant or antiplatelet drugs. However, they have the disadvantage of encountering degenerative calcific dystrophy processes (pig bioprostheses), degeneration with rupture of the cusps (pericardial prostheses) and demonstrate a higher sensitivity towards endocardial infections. These bioprostheses are usually treated with chemical agents, such as glutaraldehyde, in order to improve mechanical characteristics whilst reducing the intrinsic tissue antigenicity allowing the preservation thereof.

They can also be treated with decalcification protocols (aiming at decreasing the risk of hydroxyapatite crystal deposition following implant) or detoxification agents, through the saturation of any glutaraldehyde aldehydic sites that are still reactive. The bioprostheses, of both porcine and bovine/equine origin, have been divided into 1st generation (tissues fixed with glutaraldehyde at high pressure) and 2nd generation bioprostheses (glutaraldehyde at low pressure with the addition of surfactants blocking the calcium binding sites), both having an average duration of 10-14 years.

As previously mentioned, all biological valve prostheses (with the exception of homografts) are of xenogeneic origin. Xenogeneic tissue belongs to an organism of a species other than man; these materials possess specific surface antigens tolerable exclusively inside the same species of origin, but incompatible in the case of a human implantation where, if not adequately treated, they would trigger the activation of the complement cascade with platelet aggregation, generating a situation similar to that occurring in the case of blood group incompatibility. This reaction is known as hyperacute rejection and occurs in about thirty minutes. The main factor responsible for this dramatic rejection mechanism is the $\alpha$-Gal xenoantigen. This epitope is a di-galactoside (galactose-alpha 1,3-galactose) present on membrane glycoproteins and glycolipids, primarily of endothelial cells, even if it was also found on cell types such as monocytes, granulocytes [2] and red blood cells [3] and in important tissue districts such as the myocardial [4] and bone [5] regions. The alpha-Gal epitope is constitutively expressed in all mammals, with the exception of higher primates and humans [6] who, due to mutations occurring during the evolutionary process, are unable to synthesize the $\alpha$-1,3-galactosylltransferase enzyme, responsible for linking the epitope to the membrane glycoproteins and glycolipids of the cellular components. Furthermore, humans express antibodies directed against this antigen since birth as the result of a continuous stimulation induced by the intestinal bacterial flora (primarily *Escherichia coli,* but also *Klebsiella pneumonia* and *Serratia marcescens*) [6]. The 1-3% of all circulating immunoglobulins in human (IgG, IgA and IgM isotypes) are specifically directed towards the $\alpha$-Gal xenoantigen.

Currently, the biocompatibility of the xenogeneic tissues intended for use in the manufacture of valve bioprostheses is obtained treating devices with the aforementioned glutaraldehyde. More specifically, this chemical agent is a bifunctional aldehyde, featuring 5 carbon atoms, introduced into biomedical practice at the end of the 1960s; glutaraldehyde reacts with the $\varepsilon$-amino groups of lysine residues by inducing the formation of a network of chemical bonds aimed at stabilising the tissues, preventing the enzymatic degradation thereof and masking cell surface antigens from the recipient's immune system.

Despite this treatment, the $\alpha$-Gal epitope has been shown (only qualitatively and only with reference to the single cellular component) to remain responsive in marketed valve bioprostheses [7], causing after the implant an increase in the anti $\alpha$-Gal antibodies titer both in paediatric and adult patients [8]. In addition, the antigen-antibody complex appears to be directly involved in promoting of calcium salts deposition [9].

Recently the Inventors developed an ELISA (Enzyme Linked Immunosorbent Assay) immunological test aimed to determine the amount of $\alpha$-Gal epitopes in both fresh xenogeneic tissues and after decellularizing treatment, and to identify a procedure capable of removing any $\alpha$-Gal xenoantigens present in the native tissue [10].

The technical problem to detect $\alpha$-Gal xenoantigens in fixed tissue has been already faced in scientific literature.

Konakci KZ et al. [7] disclose a method for the determination of the $\alpha$-Gal xenoantigens comprising an incubation with vimentin/IB4 lectin conjugates and immunohistochemical detection of the bound conjugates.

McGregor CG et al. [11] also disclose a method for the determination of the $\alpha$-Gal xenoantigens comprising an incubation with *Griffonia simplicifolia-IB4* lectin conjugates but recognise the problem to bind anti $\alpha$-Gal antibodies to glutaraldehyde-fixed issues in GTKO mice.

Simionescu DT [12] and EP075337 [13] disclose that the aldehyde reactive groups of glutaraldehyde-fixed tissues could be neutralised with reactive amines, such as for example glutamine, glycine, homocysteic acid and lysine.

Nevertheless, the problem of quantitatively determining this epitope in fixed tissues, in particular with glutaraldehyde, and primarily used for the manufacture of bioprostheses, remains unsolved.

Therefore, it is a purpose of the present invention the development of a method for detecting the $\alpha$-Gal xenoantigen in fixed tissues, so as to have a control tool capable to assure and validate the production of "$\alpha$-Gal free" tissues intended for the production of bioprostheses for clinical use.

**Summary**

For the aforementioned purpose the Inventors have developed a new method for the quantification of the $\alpha$-Gal epitope in fixed tissues, making changes to the method for determining this antigen by means of the use of a monoclonal antibody (mouse produced) developed on fresh unfixed tissues.

To enable the detection of $\alpha$-Gal in fixed tissues, it is in fact necessary to inactivate the free reactive groups of the glutaraldehyde that could interact with the anti $\alpha$-Gal monoclonal antibody, generating false positives. This inactivation can be guaranteed by treating the tissue to be analysed with homocysteic acid.

In a first aspect a method for determining the alpha-Gal xenoantigen in fixed tissues, is therefore the object of the present invention, said method comprising at least the steps of:

- subjecting a sample of a fixed tissue to inactivation treatment with homocysteic acid;
- detecting the $\alpha$-Gal epitope by means of an ELISA immunoassay with a primary anti $\alpha$-Gal monoclonal antibody;

quantifying the $\alpha$-Gal epitope by means of detection by absorbance at 450nm of the signal generated by residual free amount of said monoclonal antibody and reacted with a secondary peroxidase-conjugated antibody and comparison with a calibration line.

Yet, it is a further object of the invention the kit to carry out the method for the determination of the amount of $\alpha$-Gal xenoantigen in fixed tissues to be used as bioprosthetic replacements.

The method, the possible embodiments and the advantages thereof, as well, will be clarified by the detailed description of the invention which follows and with the aid of the example embodiments thereof provided by way of a non-limiting example of the invention.

**Brief description of the drawings**

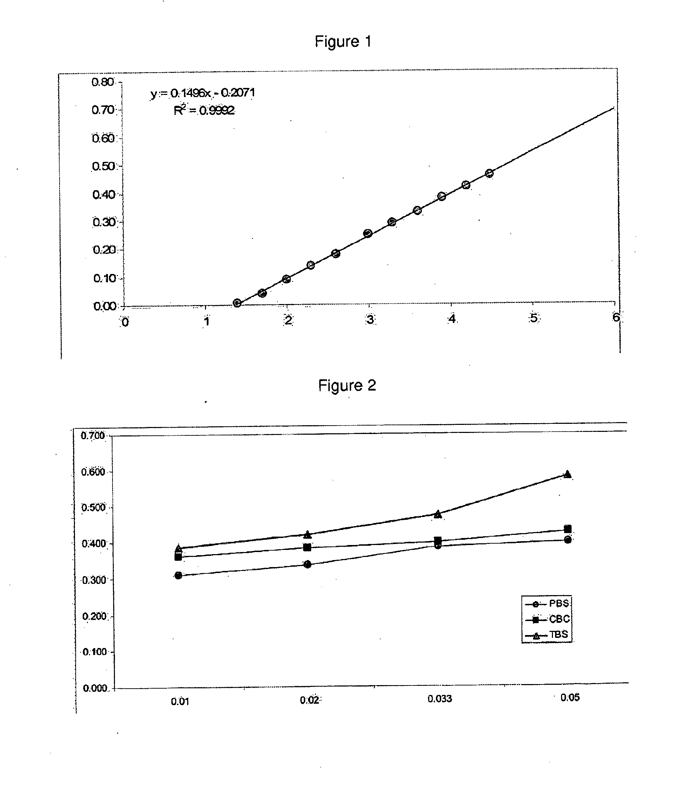

Figure 1: calibration line that correlates the different $\mu$g of reactive primary anti $\alpha$-Gal monoclonal antibody during the incubation period (the y-axis) with the logarithm of the corresponding different concentrations of rabbit red blood cells (abscissa). Each red cell, as indicated by literature, expresses $2 \times 10^6$ epitopes $\alpha$-Gal on its surface.

Figure 2 : evaluation of saturation degree of the bottom of the plate obtained through coating made with three different buffers. The x-axis shows different dilutions of the original primary anti $\alpha$-Gal monoclonal antibody: [1:100] - [1:50] - [1:30] - [1:20], the y-axis shows the relative absorbance for each previously mentioned concentration obtained by reading at 450 nm. PBS: phosphate buffered saline, CBC: carbonate/bicarbonate buffer, TBS: Tris buffer/NaCl.

**Detailed description of the invention**

The method for determining the alpha-Gal epitope according to the invention is applicable to a wide range of substitutes such as bioprosthetic devices usable in human clinical field, and in particular to substitutes such as bioprosthetic devices derived from tissues of porcine and bovine or equine pericardial origin, fixed in glutaraldehyde according to the procedures of each manufacturer.

The method of the invention, which comprises at least the steps of:

- subjecting a sample of a fixed tissue to an inactivation treatment with homocysteic acid;
- detecting the $\alpha$-Gal epitope by means of an ELISA immunoassay with a primary anti $\alpha$-Gal monoclonal antibody;
- quantifying the $\alpha$-Gal epitope by means of detection by absorbance at 450nm of the signal generated by the residual free anti $\alpha$-Gal monoclonal antibody and comparison with a calibration line,

may optionally comprise, prior to determining the $\alpha$-Gal epitope, the further step of:

- producing a calibration line by using rabbit red blood cells for the quantitative determination of the $\alpha$-Gal epitope following the detection by absorbance at 450nm of the signal of residual free anti $\alpha$-Gal monoclonal antibody signal.

The method can be carried out in one embodiment thereof according to the protocol the essential conditions of which are described below:

- preparing a sample of a glutaraldehyde-fixed tissue and treating the same for inactivation with a homocysteic acid solution in phosphate buffered saline (PBS) at a pH of between 6.0 - 8.0 and at a concentration of between 50mM and 200mM (saturation limit); the concentration of homocysteic acid is preferably 100mM;
- immunoassay by ELISA on the inactivated sample following incubation with a primary anti $\alpha$-Gal monoclonal antibody.

The primary anti $\alpha$-Gal monoclonal antibody for the ELISA assay is a monoclonal antibody that can be obtained for example in mice by immunization with rabbit red blood cells. This antibody, belonging to the IgM family, has a characteristic pentameric structure that allows the creation of immunocomplexes of large dimension that can be separated by centrifugation. The terms "monoclonal antibody" and "anti $\alpha$-Gal antibody" used herein in any case identify a monoclonal antibody directed exclusively against the $\alpha$-Gal epitope usable for the method of the invention as primary antibody in the ELISA assay step. Preferably, for the ELISA immunoassay step the primary anti $\alpha$-Gal monoclonal antibody is a mouse antibody, while the secondary antibody is a polyclonal antibody conjugated with the peroxidase enzyme and said polyclonal antibody is preferably an anti-mouse rabbit antibody.

The method, object of the invention, was developed with the anti $\alpha$-Gal monoclonal antibody commercialized by Axxora (Nottingham, UK) and identified as M86, which is preferable, although the method itself cannot be either constrained or limited to the above commercial product containing this anti $\alpha$-Gal monoclonal antibody in the implementation thereof.

In performing the method for the determination of the $\alpha$-Gal epitope, it is preferable to perform a coating of the plate (coating means covering the bottom of the wells) with 50 $\mu$l per well of $\alpha$-Gal epitope/human serum albumin (HSA) or with $\alpha$-Gal epitope/bovine serum albumin (BSA). The use of human serum albumin is however preferable as its bovine alternative, constitutively containing $\alpha$-Gal epitopes, increases the background noise observed at the time the spectrophotometric reading. These solutions ($\alpha$-Gal epitope/HSA or $\alpha$-Gal epitope/BSA) are used at a concentration comprised between 2.5 - 15 $\mu$g/ml. The concentration of 5 $\mu$g/ml is preferably used.

The following buffers can be used for the coating solution: phosphate buffered saline (PBS) [136 mM NaCl, 2 mM KCl, 4 mM $Na_2HPO_4$, 2 mM $KH_2PO_4$], pH 7.0-8.0; carbonate/bicarbonate buffer (CBC) [35 mM $NaHCO_3$, 15 mM $NaCO_3$] pH 9.0-10.0; Tris/NaCl buffer (TBS) [50 mM Tris, 0.9 % NaCl], pH 7.0-8.0. Improved saturation of the plate is however obtained with the use of the CBC and TBS buffers, which are therefore preferable to the PBS buffer. Optimal saturation is achieved with the TBS buffer, which should be thus preferably used as an alternative to CBC.

The quantification of the $\alpha$-Gal epitope is preferably not directed and is obtained calculating the anti $\alpha$-Gal monoclonal antibodies remaining in the supernatant and not reacted with the $\alpha$-Gal epitope of the fixed tissue. These free anti $\alpha$-Gal monoclonal antibodies are reacted with secondary peroxidase-conjugated antibodies and the absorbance at 450 nm of the immunocomplexes so formed are read by a spectrometer. The expressions "free monoclonal antibody signal" or "signal of free amount of anti $\alpha$-Gal monoclonal antibody" are herein used with reference to the signal deriving from these immunocomplexes.

In one preferred embodiment, the method object of the invention provides that the different steps are carried out under the conditions described in detail below.

Preparation of fixed tissue samples and treatment with homocysteic acid

Tissue samples collected from the bioprosthesis are quickly washed with sterile phosphate buffer (PBS), to eliminate the glutaraldehyde excess present in the preservation solution of the bioprosthesis, and incubated with 100 mM homocysteic acid in sterile PBS (pH 6.0 - 8.0). This incubation is performed with two steps, each of 12 hours (with replacement of the buffer with a fresh solution after the first 12 hours) under constant stirring, in the dark at room temperature, preferably comprised between 15 and 20 °C.

The sample is then washed twice, each time for 15 minutes, in sterile PBS (pH 7.0 - 8.0) at room temperature. Quickly dried on special filter paper and weighed on analytical scales.

The treatment of biological material with homocysteic acid, before the ELISA assay, is defined as the inactivation step aimed at saturating the chemical residues of the unconjugated glutaraldehyde still reactive. This procedure thus allows for the interaction between $\alpha$-Gal groups with the anti $\alpha$-Gal monoclonal antibody avoiding generation of false positives.

ELISA assay: incubation with anti $\alpha$-Gal monoclonal antibody.

Each sample is labelled and cut into small pieces weighing between 30 and 50 mg; a sample of primary anti $\alpha$-Gal monoclonal antibody in sterile PBS [1:50] v/v is added thereto and allowed to incubate under moderate agitation at 37 °C for 2 hours (final volume between 1.5 and 2.5 ml). The sample is then centrifuged at 14,340 rpm for 30 minutes at +4°C.

ELISA assay: preparing the plate wells

A coating with 50 $\mu$l per well of $\alpha$-Gal epitope/human serum albumin (HSA) at 5 $\mu$g/ml in TBS buffer (pH 7.0 - 8.0) is made on a 96-well plate. The plate is subsequently covered with protective film to prevent any phenomena of evaporation, and incubated for 90 minutes at 37°C in the dark. 3 washes were then performed with 250 $\mu$l per well of sterile PBS (pH 7.4) at room temperature. The first wash was left to rest for 5 minutes, the two subsequent washes for 3 minutes.

Blocking procedure (essential step in ELISA assays for making the plastic inert to more specifically micro-areas of the bottom of the well that may have remained free of the saturated coating procedure) is carried out with 250 $\mu$l per well of 1.5 % HSA w/v in sterile PBS, followed by covering of the plate with protective film and incubation for 2 hours at room temperature, in the dark. Further 3 washes with sterile PBS are then carried out as set out above.

Preparing the calibration curve with different concentrations of rabbit erythrocytes

The erythrocytes are isolated from whole rabbit blood by classic method, resuspended in sterile isotonic solution (pH 7.4) at 4°C and counted via a Bürker chamber. The calibration line is developed with eleven different concentrations (expressed as red blood cells no./2 ml) of red blood cells; $30.5 \times 10^6$, $16 \times 10^6$, $8 \times 10^6$, $4 \times 10^6$, $2 \times 10^6$, $1 \times 10^6$, $0.4 \times 10^6$, $0.2 \times 10^6$, $0.1 \times 10^6$, $0.05 \times 10^6$ and $0.025 \times 10^6$. Eleven test tubes containing the different concentrations (2 ml final) are then prepared and incubated with the original diluted anti $\alpha$-Gal monoclonal antibody preparation [1:50] v/v in sterile PBS for 2 hours at 37°C under constant agitation. The samples were then centrifuged at 14,340 rpm for 30 minutes at +4°C.

ELISA assay: determining the $\alpha$-Gal antigen

100 $\mu$l of the supernatant, collected from each sample to be analysed previously incubated with the anti $\alpha$-Gal monoclonal antibody and from every solution of red blood cells at different concentrations, were added for each well The plate is loaded by occupying the wells of an entire column for each sample and then the plate is covered with protective film and incubated overnight at 4°C in the dark. 3 washes with sterile PBS are then carried out as set out above. 100 $\mu$l per well of a secondary antibody solution (anti-mouse rabbit polyclonal) conjugated to the peroxidase enzyme [1:500] v/v in sterile PBS are added; the plate is then covered with protective film and incubated at 37°C in the dark for 2 hours. 3 washes with sterile PBS are carried out as above. 100 $\mu$l per well of a developer solution for the peroxidase enzyme (o-phenylenediamine 200 $\mu$moles in phosphate-citrate buffer and sodium perborate) are added; the addition is followed by the covering of the plate with protective film, and by the incubation for 30 minutes in the dark. The plate is then read in a plate reader at 450 nm to detect the free antibody amount.

The assay developed is of the indirect type: the sample to be analysed is incubated with a known amount of primary anti $\alpha$-Gal monoclonal antibody (monoclonal antibody directed exclusively against the $\alpha$-Gal epitope). The immunocomplexes created were separated by centrifugation. Dosage of the primary anti $\alpha$-Gal monoclonal antibodies remaining free in solution (namely, not reacted with the $\alpha$-Gal epitope of the fixed tissue) and reacted with the peroxidase-conjugated secondary antibodies is then performed. The difference allows the amount of antibody bound to the antigen $\alpha$-Gal epitope to be calculated. The quantitative determination of the $\alpha$-Gal epitopes is performed by comparison of a known amount of $\alpha$-Gal epitopes deriving from a standard source consisting of rabbit red blood cells ($2 \times 10^6$ epitopes/red blood cell), and the amount detected in the bioprostheses.

The conversion formula for calculation of the number of $\alpha$-Gal epitopes starting from the analysis of a biological sample was extrapolated from the averages obtained from the construction of 3 calibration curves with eleven different rabbit red blood cell concentrations each carried out in triplicate.

The resulting mathematical formula is:

$$\text{number of epitopes} \ = \ 10^{\left(\frac{\text{ug AbI} + 0.2071}{0.1496}\right)} * 2 * 10^{9}$$

where:

- $\mu$G AbI corresponds to micrograms of primary monoclonal antibody (ABI) having bound to the sample during 2 hours of incubation at 37°C in the dark. This amount can be determined through a comparison between the absorbance generated by a known amount of anti $\alpha$-Gal monoclonal antibody before and after incubation with the sample knowing that every $\mu$l of stock antibody solution used contains 0.015 $\mu$l of monoclonal antibody. The formula was extrapolated from the calibration line provided in Figure 1, obtained as previously described, and it is to be considered applicable for antibody amounts ranging from 0.005 $\mu$g to 0.47 $\mu$g. Therefore, in one embodiment, the method of the present invention can comprise the steps of:

- treating a tissue sample by incubating with homocysteic acid solution in a buffer for 12 hours x 2, under agitation in the dark and at room temperature;
- incubating the sample previously treated with a primary anti $\alpha$-Gal monoclonal antibody for 2 hours at 37°C;
- centrifuging the sample at 14,340 rpm for 30 minutes at 4°C and separating the supernatant;
- treating a well plate for coating with a buffered solution of $\alpha$-Gal epitope/serum albumin;
- washing the wells with a buffer and treating them for blocking with a buffered solution of 1.5% w/v serum albumin for 2 hours at room temperature;
- washing the wells with the buffer, loading the supernatant of the previously centrifuged sample and incubating overnight at 4°C;
- washing the wells with buffer and loading the secondary polyclonal anti-mouse antibody conjugated to the peroxidase enzyme and incubating overnight at 4°C;
- washing with buffer, loading the wells with the developer solution and incubating in the dark for 30 minutes;
- reading the plate with a plate reader at 450 nm for the detection by absorbance of the free monoclonal antibody signal.

In one preferred embodiment thereof, this method for determining the $\alpha$-Gal xenoantigen in glutaraldehyde-fixed tissues comprises the following steps:

- treating the tissue sample to be analysed by incubating with homocysteic acid solution preferentially at the concentration of 100 mM, pH 6.0-8.0, in a phosphate buffer for 12 hours x 2, under agitation, in the dark and at room temperature;
- incubating the sample previously treated with a primary anti $\alpha$-Gal monoclonal antibody for 2 hours at 37°C;
- centrifuging the sample at 14,340 rpm for 30 minutes at 4°C and separating the supernatant;
- coating a well plate with 50 $\mu$l per well of $\alpha$-Gal epitope/human serum albumin (HSA) at 5 $\mu$g/ml in TBS buffer (pH 7.0 - 8.0);
- washing the wells with PBS and blocking with 1.5 % HSA in PBS for 2 hours at room temperature;
- washing the wells with PBS and 100 $\mu$l load of the supernatant of the previously centrifuged sample per well and incubating overnight at 4°C;
- washing the wells with buffer and loading the secondary polyclonal anti-mouse antibody conjugated to the peroxidase enzyme and incubating overnight at 4°C;
- washing with PBS and 100 $\mu$l load of the developer solution and incubation in the dark for 30 minutes;
- reading of the plate with a plate reader at 450 nm for the detection by absorbance of the free monoclonal antibody signal.

The enablement of the method for determining the alpha-Gal epitope to glutaraldehyde-fixed tissues, capable of assuring and validating the production of "$\alpha$-Gal free" tissues intended for the production of bioprostheses for clinical use, requires the identification of conditions that will not result in false positives/negatives. With respect to the method applicable to fresh tissues the substantial differences are:

- the development of a new method that foresees the use of homocysteic acid at a preferable concentration of 100 mM and at pH 6.0 - 8.0, for saturation of the glutaraldehyde groups capable of generating false positives following interaction with a primary anti $\alpha$-Gal monoclonal antibody;
- the use of $\alpha$-Gal epitope /HSA for the coating solution to increase epitope detection sensitivity through the elimination of background noise;
- the use of a new solution of coating consisting of buffer TBS able to increase the efficiency of $\alpha$-Gal epitope /HSA at the bottom of the plate at any concentrations of primary antibody monoclonal anti $\alpha$-Gal (see Figure 2);
- the use of a new solution of coating consisting of buffer CBC able to increase the efficiency of $\alpha$-Gal epitope /HSA at the bottom of the plate for antibody concentrations of primary antibody monoclonal anti $\alpha$-Gal up to [1:50] v/v (see Figure 2).

In a second aspect, the invention relates to a kit aimed at performing the method for the determination of the $\alpha$-Gal epitope in tissues fixed with glutaraldehyde that comprises at least:

- at least one first container for a homocysteic acid solution;
- at least one second container for a primary anti $\alpha$-Gal monoclonal antibody;
- at least one further container for a secondary peroxidase-conjugated polyclonal antibody, preferably an anti-mouse rabbit antibody;
- instruction leaflet detailing the calibration line arranged with known concentrations of rabbit red blood cells and the conversion formula for calculating the number of $\alpha$-Gal epitopes in the analysed tissue on the basis of the sample absorbance read at 450 nm.

The kit can preferably further comprise:

- at least one container with a coating buffer consisting of an $\alpha$-Gal epitope/serum albumin solution in a buffer;
- at least one container with a blocking buffer consisting of a serum albumin solution in a buffer;
- at least one container with a solution for the development of peroxidase enzyme.

The kit intended use is the quality control of all those biological tissues fixed (with glutaraldehyde for example) which can be used for the manufacture of prostheses applicable in human clinical use.

As will become apparent in the reported examples below to evaluate the effective action of the treatment with homocysteic acid, knock-out porcine tissue for the antigen $\alpha$-Gal (the gene responsible for epitope expression is silenced through a genetic modification) were used. This non-fixed tissue, incubated with the anti $\alpha$-Gal monoclonal antibody, does not generate any signal as it is missing the antigen.

Performing the assay on the same tissue following glutaraldehyde fixation, positive and extremely variable results (false positives) are generated. Following treatment with homocysteic acid, the results obtained from the knock-out glutaraldehyde-fixed tissue are comparable to those of the original non-fixed tissue, specifically there is no signal on the part of the anti $\alpha$-Gal monoclonal antibody.

The opportunity to apply this assay to fixed biological prosthetic replacements provides a screening tool that gives preference to products that are able to demonstrate or that will demonstrate the achieving of a complete blocking or inactivation of the $\alpha$-Gal xenoantigen. These bioprostheses can ensure a better biocompatibility and a decreased propensity for the onset of inflammation and calcific dystrophy phenomena.

Currently, xenogeneic tissue patches are used to reconstruct and repair tissue districts not limited to the scope of cardiac valves; for this purpose, it is useful to cite CorMatrix® (patch for interventions and reconstructions in the cardiovascular field), Dynamatrix® (for interventions in the dental field), SurgiSIS® Ophthalmics (used in the context of the ocular surgery), AxoGuard® Nerve Connector (used in the context of surgical nerve connection), Matristem® (used in the ulcer repairing). The method object of the invention can thus find application as a quality control for all of the xenogeneic tissues used in the context of tissue engineering and not only in the cardiac valve field as valve replacements.

EXAMPLES

In all the below examples the ELISA assay was performed with the primary $\alpha$-Gal monoclonal antibody marketed by Axxora (Nottingham, UK) identified as M86.

*Example 1: quantitative detection of the $\alpha$-Gal epitope in a fresh knock-out $\alpha$-Gal porcine tissue.*

The knock-out pericardium was transported to a laboratory in a cold isotonic solution (PBS, 4°C at pH 7.4) added with 1% antibiotics (penicillin and streptomycin). All the operations performed on the sample were carried out while ensuring sterility conditions. In the laboratory the pericardial sac was laid out on a plane and the lipid component was mechanically separated from the epipericardial surface, then the tissue was subjected to a short wash in sterile phosphate buffered saline (PBS, 4°C at pH 7.0 - 8.0). The pericardial sac was cut into smaller samples weighing about 40 mg, which are each subject to two 15 minute washings in PBS to remove any blood residues.

Then each sample (n= 6) was subjected to a filter paper blotting and accurately weighed, before being subjected to the ELISA test. This sample was designated as "KO porcine pericardium".

The results achieved are recorded in the following table 1.

*Example 2: quantitative detection the $\alpha$-Gal epitope in an $\alpha$-Gal knock-out glutaraldehyde-fixed porcine tissue.*

The knock-out pericardium was transported to the laboratory in a cold isotonic solution (PBS, 4°C at pH 7.0 - 8.0) added with 1% antibiotics (penicillin and streptomycin). All the operations performed on the sample were carried out while ensuring sterility. In the laboratory the pericardial sac was laid out on a plane and the lipid component was mechanically separated from the epipericardial surface, then the tissue was subjected to a short PBS wash. The pericardial sac was cut into smaller samples from weighing about 40 mg, which are each subject to two steps of washings for 15 minutes each in PBS in order to remove any blood residues.

Glutaraldehyde fixation took place according to the procedure illustrated by Stacchino C. and colleagues [14], which envisages incubations each having a three-day duration in an increasing concentration of glutaraldehyde up to 05% (3 days of incubation for each concentration step 0.1%, 0.2%, 0.3%, 0.4%, 0.5%). Each sample (n= 6) was subsequently subjected to a filter paper blotting and accurately weighed, before being subjected to the ELISA test. This sample was designated as "KO F porcine pericardium".

Following the glutaraldehyde fixation procedure, some of the fixed samples were subjected to inactivation treatment. They were then incubated with homocysteic acid at a concentration of 100 mM in phosphate buffer for 12 hours x 2 under stirring, in the dark at room temperature. Each sample (n= 6) was subsequently subjected to a filter paper blotting and accurately weighed, before being subjected to the ELISA test. This sample was designated as "KO F-DETOX porcine pericardium".

The results obtained from determination of the $\alpha$-Gal epitope in examples 1 and 2 are shown in table 1, expressed as the number of $\alpha$-Gal epitopes $\times 10^{10}$ /10 mg of wet tissue in $\alpha$-Gal-knock-out porcine pericardium (KO, n=6), $\alpha$-Gal-

knock-out porcine pericardium following only glutaraldehyde fixation (KO F, n= 6) and $\alpha$-Gal -knock-out porcine pericardium following glutaraldehyde fixation and inactivation (KO F-DETOX, n= 6).

Table1

| | Number of epitopes $\times 10^{10}$ Every 10 mg of wet tissue |
|---|---|
| KO porcine pericardium | $0.0001 \pm 0.15$ |
| KO F Pericardium | $1.56 \pm 0.95$ |
| KO F-DETOX porcine pericardium | $0.001 \pm 0.4$ |

Results show that the inactivating treatment with homocysteic acid under the conditions indicated is a crucial step in order to carry out a reliable and reproducible survey aimed at determining the $\alpha$-Gal epitope on tissue samples previously fixed in glutaraldehyde.

*Example 3: quantitative detection of the $\alpha$-Gal epitope in commercial aortic valve bioprostheses obtained from glutaraldehyde-fixed porcine or bovine tissue.*

All handling of bioprosthetic materials took place while maintaining sterility. Each bioprosthesis was extracted from the relevant package and subjected to 3 washes in phosphate buffered saline (PBS, 20°C at pH 7.0 - 8.0) as expressly indicated by the Pre-implantation Instruction Manual provided by the manufacturer. The leaflets were then isolated and subjected to inactivating treatment by means of incubation with homocysteic acid at a concentration of 100 mM in phosphate buffer for 12 hours x 2 under stirring, in the dark at room temperature. Each sample was then subjected to a blotting on filter paper and accurately weighed, before being subjected to the ELISA test.

The results expressed as the mean $\pm$ standard deviation of the number of epitopes $\times 10^{10}$/10 mg of wet tissue are reported in table 2. The statistical significance between samples was determined by means of the one way ANOVA test. Differences were considered significant with $p < 0.05$ .

Table 2

| BIOPROSTHESIS | ORIGIN | NUMBER OF EPITOPES |
|---|---|---|
| model 1 n=6 | bovine pericardium | $6.62*10^{10} \pm 0.43$ |
| model 2 n=3 | bovine pericardium | $5.9*10^{10} \pm 0.7$ |
| model 3 (TAVI*) n=3 | bovine pericardium | $6.3*10^{10} \pm 0.7$ |
| model 4 n=3 | bovine pericardium | $5.2*10^{10} \pm 0.67$ |
| model 5 n=9 | porcine valve | $5.02*10^{10} \pm 0.68$ |

*TAVI (Transcatheter Aortic Valve Implantation) percutaneous or trans apically implantable aortic prosthesis.

*Example 4: evaluation of the buffer of coating in guaranteeing improved binding of the serum protein with the bottom of the plate.*

The molecule responsible for the binding between the plastic forming the bottom of the wells and the $\alpha$-Gal residue of the epitope is serum albumin (whether human or bovine). The binding occurs through charge interactions between the plastic and the protein itself. The buffer in which the coating solution is dissolved is able to influence the protein charge.

An assessment of 3 different buffers was therefore carried out in order to highlight which of the buffers was more suitable in preparing the serum protein to bind the plate. $\alpha$-Gal epitope /HSA and $\alpha$-Gal epitope /BSA were dissolved at a concentration of 5 $\mu$g/ml in the three different buffers:

a) phosphate buffer (PBS) [136 mM NaCl, 2 mM KCl, 4 mM $Na_2HPO_4$, 2 mM $KH_2PO_4$] pH 7.0 - 8.0;

b) carbonate/bicarbonate buffer (CBC) [35 mM $NaHCO_3$, 15 mM $NaCO_3$] pH 9.0 - 10.0;

c) Tris buffer/NaCl buffer (TBS) [50 mM Tris, 0.9 % NaCl] pH 7.0 - 8.0.

A better arrangement of the serum protein, translates into a greater presence of $\alpha$-Gal residues on the bottom of the plate. An increase in the number of these residues can be seen through increasing concentrations of primary anti $\alpha$-Gal M86 monoclonal antibody.

A coating was thus carried out (according to the above-described procedures) of three plates each with a different buffer. Each plate was subsequently covered with protective film to prevent any evaporation phenomena, and incubated for 90 minutes at 37°C in the dark.

3 washes were carried out with 250 $\mu$l per well of sterile PBS at room temperature. The first wash was left to rest for 5 minutes, the next two for 3 minutes.

The blocking was carried out with 250 $\mu$l per well of 1.5 % HSA in sterile PBS (pH 7.4), followed by covering of the plate with protective film and incubation for 2 hours at room temperature, in the dark. 3 washes with sterile PBS were subsequently carried out as above.

100 $\mu$l per well of four different concentrations of primary M86 antibody in PBS divided by columns were added to each plate containing a different buffer: [1:100], [1:50], [1:30], [1:20] v/v. The plates were then incubated overnight at +4°C in the dark. 3 washes with sterile PBS were then carried out as set out above.

100$\mu$l of secondary antibody solution (polyclonal from anti-mouse rabbit) conjugated to the peroxidase enzyme at a concentration of [1:500] v/v in sterile PBS were added to each well. The plate was then covered with protective film and incubated at 37°C in the dark for 2 hours. 3 washes with sterile PBS were carried out as set out above.

Addition of 100 $\mu$l per well of the developer solution for the peroxidase enzyme (o-phenylenediamine 200 $\mu$moles in phosphate-citrate buffer and sodium perborate), covering the plate with protective film and incubation for 30 minutes in the dark were performed.

The plate reading was carried out in a plate reader at 450 nm.

The results are shown in Figure 2. The above figure shows how the CBC buffer guarantees a serum protein binding yield and therefore presence of $\alpha$-Gal residues of the epitope on the bottom of the best plate of the PBS for antibody concentrations ranging from 1:100 to 1:50. At higher concentrations the actions of the CBC and PBS buffers are superimposable.

The TBS buffer, on the other hand, has been proved to prepare the serum protein at an optimum and longer-lasting binding with the bottom of the wells even for high antibody concentrations such as [1:30] and [1:20] v/v.

All buffers are satisfactory by substantially using for the M86 antibody concentration equal to [1:50] v/v for performance of the method according to the invention. It is however preferable to use the TBS buffer for the marked adjuvant properties of the bond between the serum protein and the plastic of the plates used.

## References

1. Nkomo VT, Gardin JM, Skeleton TN, Gottdiener JS, Scott CG, Enriquez-Sarano M. Burden of valvular heart diseases: a population-based study. Lancet 2006; 368: 1005-1011.

2. Zeng LY et al. A prompt method to quantitative assay of alpha-Gal on pig cell surface without injury. Sichuan Da XUe Xue Bao Yi Xue Ban. 2005; 36(3): 419-421.

3. Galili U et al. A sensitive assay for measuring alpha Gal epitope expression on cells by a monoclonal anti-Gal antibody. 2005; 1998(65): 1129-1132.

4. Chen RH, Kadner A, Mitchell RN, Santerre DH, Adams DH. alpha-Gal and beta-Gal are preferentially expressed on porcine cardiac microvascular endothelium. Transplant Proc 2000; 32 877-878.

5. Feng W et al. Distribution of the Alpha-Gal epitope on adult porcine bone tissue. Transplant. Proceed. 200638. 2247-2251.

6. Galili U. The alpha Gal epitope and the anti-Gal antibody in xenotransplantation and in cancer immunotherapy. Immun. Cell Biol. 2005; 83: 674-686.

7. Konakci KZ, Bohle B, Blumer R, Hoetzenecker W, Roth G, Moser B, Boltz-Nitulescu G, Gorlitzer M, Klepetko W, Wolner E, Ankersmit HJ. Alpha-Gal on bioprostheses: xenograft immune response in cardiac surgery. Eur J Clin Invest. 2005 Jan; 35(1): 17-23.

8. Park CS, Park SS, Choi SY, Yoon SH, Kim WH, Kim YJ. Anti alpha-gal immune response following porcine bioprosthesis implantation in children. J Heart Valve Dis. 2010 Jan; 19(1): 124-130.

9. Lila N, McGregor CG, Carpentier S, Rancic J, Byrne GW, Carpentier A. Gal knock-out pig pericardium: new source of material for heart valve bioprostheses. J Heart Lung Transplant. 2010 May; 29(5): 538-543. Epub 2009 Dec 29.

10. Naso F, Gandaglia A, Iop L, Spina M, Gerosa G. First quantitative assay of alpha-Gal in soft tissues: presence and distribution of the epitope before and after cell removal from xenogeneic heart valves. Acta Biomater. 2011 Apr; 7(4): 1728-1734. Epub 2010 Nov 29.

11. Mc Gregor CGA, Carpentier A, Lila N, Logan JS and Byrne GW. Cardiac xenotransplantation technology provides materials for improbe bioprosthetic heart valves. J. Thos. Card. Surg., 2011, 141 (1), 269-275.

12. Simionescu DT. Artificial Heart Valves, Wiley Encyclopedia of Biomedical Engineering, John Wiley & Sons Inc., 2006, 1-10.

13. Sorin Biomedica Cardio SPA, A method of preparing biological implantation material, EP 0 795 337, 17 September 1997.

14. Stacchino C, Bona G, Bonetti F, Rinaldi S, Della Ciana L, Grignani A., Detoxification process for glutaraldehyde-treated bovine pericardium: biological, chimica and mechanical characterization. J Heart Vale Dis. 1998 Mar: 7(2): 190-194.

## Claims

1. A method for determining α-Gal xenoantigen in glutaraldehyde-fixed tissues comprising at least the steps of:

   - subjecting a sample of a fixed tissue to an inactivation treatment with homocysteic acid;
   - detecting α-Gal epitope by means of an ELISA immunoassay with a primary anti α-Gal monoclonal antibody;
   - quantifying the α-Gal epitope by means of detection by absorbance at 450 nm of the signal generated by free amount of said anti α-Gal monoclonal antibody reacted with a secondary peroxidase-conjugated antibody and comparison with a calibration line.

2. The method for determining α-Gal xenoantigen in glutaraldehyde-fixed tissues according to claim 1, further comprising the step of:

   - producing the calibration line by using rabbit red blood cells for the quantitative determination of α-Gal epitope following the detection by absorbance at 450 nm of the signal of free amount of said anti α-Gal monoclonal antibody.

3. The method for determining α-Gal xenoantigen in glutaraldehyde-fixed tissues according to claim 1, wherein for the inactivation treatment the homocysteic acid is in a phosphate buffer with a pH between 6.0 and 8.0 at a concentration comprised from 50 mM to 200 mM.

4. The method for determining α-Gal xenoantigen in glutaraldehyde-fixed tissues according to claim 3, wherein the homocysteic acid is at the concentration of 100 mM.

5. The method for determining α-Gal xenoantigen in glutaraldehyde-fixed tissues according to claim 1, wherein for the ELISA assay the plate coating is performed with solutions of α-Gal epitope/serum albumin at a concentration comprised from 2.5 to 15 μg/ml in a buffer selected from phosphate pH 7.0 - 8.0, carbonate/bicarbonate pH 9.0 - 1.0, and Tris/NaCl pH 7.0 - 8.0 buffers.

6. The method for determining α-Gal xenoantigen in glutaraldehyde-fixed tissues according to claim 5, wherein the plate coating is carried out with solutions of α-Gal epitope/human serum albumin in a concentration of 5 μg/ml in Tris/NaCl buffer.

7. The method for determining α-Gal xenoantigen in glutaraldehyde-fixed tissues according to claim 1, comprising the steps of:

   - treating a tissue sample by incubation the same twice with a homocysteic acid buffered solution for 12 hours, under stirring, in the dark and at room temperature;
   - incubating the previously treated sample with a primary anti α-Gal monoclonal antibody for 2 hours at 37°C;
   - centrifuging the sample at 14,340 rpm for 30 minutes at 4°C and separating the supernatant;
   - treating a well plate for coating with a buffered solution of α-Gal epitope/serum albumin;
   - washing the wells with buffer and treating for blocking with a buffered 1.5% w/v solution of serum albumin for 2 hours at room temperature;

- washing the wells with buffer, loading the supernatant of the previously centrifuged sample and incubating overnight at 4° C;
- washing the wells with buffer, loading a peroxidase-conjugated secondary polyclonal antibody and incubating overnight at 4° C;
- washing with buffer, loading the wells with a developer solution and incubating in the dark for 30 minutes;
- reading the plate with a plate reader for the detection of said free anti alpha-Gal monoclonal antibody signal by absorbance at 450 nm.

8. A kit for carrying out the method according to one of claims 1 to 7 comprising at least:

- at least one first container containing a homocysteic acid buffered solution;
- at least one second container containing a primary $\alpha$-Gal monoclonal antibody;
- at least one further container containing a secondary polyclonal antibody conjugated with the peroxidase enzyme;
- an instruction leaflet showing a calibration line arranged with known concentrations of rabbit red blood cells and the conversion formula for calculating the number of $\alpha$-Gal epitopes in the analysed tissue on the basis of the sample absorbance read in a plate at 450 nm.

9. The kit according to claim 8, further comprising:

- at least one container with a coating buffer consisting of an $\alpha$-Gal epitope/serum albumin buffered solution;
- at least one container with a blocking buffer consisting of a serum albumin buffered solution;
- at least one container with a solution for the development of peroxidase enzyme.

10. Use of the method according to one of claims 1 to 7 for the quality control of the biological fixed tissues usable for manufacturing bioprostheses of human clinical use.

## Patentansprüche

1. Verfahren zum Bestimmen von $\alpha$-Gal-Xenoantigen in mit Glutaraldehyd fixierten Geweben umfassend wenigstens die Schritte:

- Unterwerfen einer Probe eines fixierten Gewebes gegenüber einer Inaktivierungsbehandlung mit Homocysteinsäure,
- Detektieren von $\alpha$-Gal-Epitop durch einen ELISA-Immunoassay mit einem primären monoklonalen Anti-$\alpha$-Gal- ntikörper,
- Quantifizieren des $\alpha$-Gal-Epitops mittels Detektion durch Absorption bei 450 nm des Signals, welches durch die Menge an freiem, mit einem sekundären mit Peroxidase konjugierten Antikörper reagierten monoklonalen Anti-$\alpha$-Gal-Antikörper erzeugt wird, und Vergleich mit einer Kalibrierungslinie.

2. Verfahren zum Bestimmen von $\alpha$-Gal-Xenoantigen in mit Glutaraldehyd fixierten Gewerben nach Anspruch 1, welches des Weiteren den Schritt umfasst:

- Herstellen einer Kalibrierungslinie durch Verwendung von roten Blutzellen aus Kaninchen für die quanititative Bestimmung eines $\alpha$-Gal-Epitops gefolgt von der Detektion durch Absorption bei 450 nm des Signals der Menge des freien monoklonalen Anti-$\alpha$-Gal-Antikörpers.

3. Verfahren zum Bestimmen von $\alpha$-Gal-Xenoantigen in mit Glutaraldehyd fixierten Gewerben nach Anspruch 1, wobei sich die Homocysteinsäure für die Inaktivierungsbehandlung in einem Phosphatpuffer mit einem pH-Wert zwischen 6,0 und 8,0 bei einer Konzentration zwischen 50 mM und 200 mM befindet.

4. Verfahren zum Bestimmen von $\alpha$-Gal-Xenoantigen in mit Glutaraldehyd fixierten Gewerben nach Anspruch 3, wobei die Homocysteinsäure in einer Konzentration von 100 mM vorliegt.

5. Verfahren zum Bestimmen von $\alpha$-Gal-Xenoantigen in mit Glutaraldehyd fixierten Gewerben nach Anspruch 1, wobei die Plattenbeschichtung für den ELISA-Assay mit Lösungen von $\alpha$-Gal-Epitop-/Serumalbumin bei einer Konzentration zwischen 2,5 und 15 $\mu$g/ml in einem Puffer ausgewählt aus Phosphat pH 7,0 bis 8,0, Carbonat/Bicarbonat pH 9,0 bis 1,0 und Tris/NaCl pH 7,0 bis 8,0 Puffer durchgeführt wird.

6. Verfahren zum Bestimmen von α-Gal-Xenoantigen in mit Glutaraldehyd fixierten Gewerben nach Anspruch 5, wobei die Plattenbeschichtung mit Lösungen von α-Gal-Epitop/humanem Serumalbumin in einer Konzentration von 5 μg/ml in Tris/NaCl-Puffer durchgeführt wird.

7. Verfahren zum Bestimmen von α-Gal-Xenoantigen in mit Glutaraldehyd fixierten Gewerben nach Anspruch 1, welches die folgenden Schritte umfasst:

- Behandeln einer Gewerbeprobe durch Inkubation derselben zweimal mit einer gepufferten Homocysteinsäure-Lösung für 12 Stunden under Rühren im Dunkeln und bei Raumtemperatur,
- Inkubieren der zuvor behandelten Probe mit einem primären monoklonalen Anti-α-Gal-Antikörper für 2 Stunden bei 37°C,
- Zentrifugieren der Probe bei 14.340 UpM für 30 Minuten bei 4°C und Abtrennen des Überstandes,
- Behandeln einer Schachtplatte zum Beschichten mit einer gepufferten Lösung von α-Gal-Epitop/Serumalbumin,
- Waschen der Schächte mit Puffer und Behandeln mit einer gepufferten 1,5 % (Gew./Vol.)-Lösung von Serumalbumin für 2 Stunden bei Raumtemperatur zum Blockieren,
- Waschen der Schächte mit Puffer, Beladen des Überstandes der zuvor zentrifugierten Probe und Inkubieren über Nacht bei 4°C,
- Waschen der Schächte mit Puffer, Beladen eines mit Peroxidase konjugierten sekundären polyklonalen Antiköpern und Inkubieren über Nacht bei 4°C,
- Waschen mit Puffer, Beladen der Schächte mit einer Entwicklungslösung und Inkubieren im Dunklen für 30 Minuten,
- Lesen der Platten mit einem Plattenlesegerät zur Detektion des freien monoklonalen Anti-alpha-Gal-Antikörpersignals durch Absorption bei 450 nm.

8. Kit zum Durchführen eines Verfahrens nach einem der Ansprüche 1 bis 7, welches wenigstens umfasst:

- wenigstens einen ersten Behälter, welcher eine gepufferte Homocysteinsäure-Lösung enthält,
- wenigstens einen zweiten Behälter, welcher einen primären monoklonalen α-Gal-Antikörper enthält,
- wenigstens einen weiteren Behälter, welche einen sekundären mit dem Peroxidaseenzym konjugierten polyklonalen Antikörper enthält,
- ein Instruktionsblatt, welches eine Kalibierungslinie zeigt, welche mit bekannten Konzentrationen von roten Blutkörperzellen aus Kaninchen angeordnet ist und die Umrechnungsformel zum Berechnen der Anzahl von α-Gal-Epitopen in dem analysierten Gewerbe auf Basis der Probenabsorption, welche in einer Platte bei 450 nm gelesen worden ist.

9. Kit nach Anspruch 8, welches des Weiteren enthält:

- wenigstens einen Behälter mit einer Beschichtungslösung bestehend aus einer gepufferten α-Gal-Epitop/Serumalbumin-Lösung,
- wenigstens einen Behälter mit einem Blockierungspuffer bestehend aus einem gepufferten Serumalbumin-Lösung,
- wenigstens einen Behälter mit einer Lösung zur Entwicklung von Peroxidaseenzym.

10. Verwendung eines Verfahrens nach einem der Ansprüche 1 bis 7 zur Qualitätskontrolle der biologisch fixierten Gewerbe, welche zur Herstellung von Bioprothesen für humane Klinikverwendung einsetzbar sind.

**Revendications**

1. Procédé de détermination d'un xénoantigène α-Gal dans des tissus fixés au glutaraldéhyde, comprenant au moins les étapes suivantes :

- la soumission d'un échantillon de tissu fixé à un traitement d'inactivation avec de l'acide homocystéique ;
- la détection de l'épitope α-Gal au moyen d'un essai immunologique ELISA avec un anticorps monoclonal anti-α-Gal primaire ;

- la quantification de l'épitope α-Gal par détection par absorbance à 450 nm du signal généré par la quantité libre dudit anticorps monoclonal anti-α-Gal ayant réagi avec un anticorps secondaire conjugué à la peroxydase et comparaison avec une courbe d'étalonnage.

2. Procédé de détermination d'un xénoantigène α-Gal dans des tissus fixés au glutaraldéhyde selon la revendication 1, comprenant en outre l'étape suivante :

- la production de la courbe d'étalonnage en utilisant des érythrocytes de lapin pour la détermination quantitative de l'épitope α-Gal après la détection par absorbance à 450 nm du signal de la quantité libre dudit anticorps monoclonal anti-α-Gal.

3. Procédé de détermination d'un xénoantigène α-Gal dans des tissus fixés au glutaraldéhyde selon la revendication 1, dans lequel, pour le traitement d'inactivation, l'acide homocystéique est dans un tampon au phosphate ayant un pH entre 6,0 et 8,0 à une concentration de 50 mM à 200 mM.

4. Procédé de détermination d'un xénoantigène α-Gal dans des tissus fixés au glutaraldéhyde selon la revendication 3, dans lequel l'acide homocystéique est à la concentration de 100 mM.

5. Procédé de détermination d'un xénoantigène α-Gal dans des tissus fixés au glutaraldéhyde selon la revendication 1, dans lequel, pour l'essai ELISA, le revêtement de la plaque est réalisé avec des solutions d'épitope α-Gal/sérumalbumine à une concentration de 2,5 μg/ml à 15 μg/ml dans un tampon choisi parmi un tampon au phosphate pH 7,0 - 8,0, un tampon au carbonate/bicarbonate pH 9,0 - 1,0 et un tampon au Tris/NaCl pH 7,00 - 8,0.

6. Procédé de détermination d'un xénoantigène α-Gal dans des tissus fixés au glutaraldéhyde selon la revendication 5, dans lequel le revêtement de la plaque est réalisé avec des solutions d'épitope α-Gal/sérumalbumine humaine à une concentration de 5 μg/ml dans un tampon au Tris/NaCl.

7. Procédé de détermination d'un xénoantigène α-Gal dans des tissus fixés au glutaraldéhyde selon la revendication 1, comprenant les étapes suivantes :

- le traitement d'un échantillon de tissu en l'incubant deux fois dans une solution tamponnée d'acide homocystéique pendant 12 heures sous agitation, à l'obscurité et à température ambiante ;
- l'incubation de l'échantillon précédemment traité avec un anticorps monoclonal anti-α-Gal primaire pendant 2 heures à 37 °C ;
- la centrifugation de l'échantillon à 14 340 tours/minute pendant 30 minutes à 4 °C et la séparation du surnageant ;
- le traitement d'une plaque à puits pour la revêtir avec une solution tamponnée d'épitope α-Gal/ sérumalbumine ;
- le lavage des puits avec un tampon et la mise en oeuvre d'un traitement de blocage avec une solution tamponnée de sérumalbumine à 1,5 % en poids/volume pendant 2 heures à température ambiante ;
- le lavage des puits avec un tampon, le chargement du surnageant de l'échantillon précédemment centrifugé et la mise en oeuvre d'une incubation pendant une nuit à 4 °C ;
- le lavage des puits avec un tampon, le chargement d'un anticorps polyclonal secondaire conjugué à la peroxydase et la mise en oeuvre d'une incubation pendant une nuit à 4 °C ;
- le lavage avec un tampon, le chargement des puits avec une solution de révélateur et la mise en oeuvre d'une incubation à l'obscurité pendant 30 minutes ;
- la lecture de la plaque avec un lecteur de plaques afin de détecter ledit signal de l'anticorps monoclonal anti-alpha-Gal libre par absorbance à 450 nm.

8. Kit permettant de mettre en oeuvre le procédé selon l'une quelconque des revendications 1 à 7, comprenant au moins :

- au moins un premier récipient contenant une solution tamponnée d'acide homocystéique ;
- au moins un deuxième récipient contenant un anticorps monoclonal anti-α-Gal primaire ;
- au moins un récipient supplémentaire contenant un anticorps polyclonal secondaire conjugué à l'enzyme peroxydase ;
- une notice d'instruction présentant une courbe d'étalonnage agencée avec des concentrations connues d'érythrocytes de lapin et la formule de conversion permettant de calculer le nombre d'épitopes α-Gal dans le tissu analysé sur la base de l'absorbance de l'échantillon lue dans une plaque à 450 nm.

9. Kit selon la revendication 8, comprenant en outre :

   - au moins un récipient contenant un tampon de revêtement consistant en une solution tamponnée d'épitope $\alpha$-Gal/sérumalbumine ;
   - au moins un récipient contenant un tampon de blocage consistant en une solution tamponnée de sérumalbumine ;
   - au moins un récipient contenant une solution permettant le développement de l'enzyme peroxydase.

10. Utilisation du procédé selon l'une quelconque des revendications 1 à 7, pour le contrôle qualité de tissus biologiques fixés utilisables pour fabriquer des bioprothèses destinées à un usage clinique chez l'humain.

Figure 1

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 075337 A

- EP 0795337 A

**Non-patent literature cited in the description**

- **NKOMO VT ; GARDIN JM ; SKELETON TN ; GOTTDIENER JS ; SCOTT CG ; ENRIQUEZ-SARANO M.** Burden of valvular heart diseases: a population-based study. *Lancet,* 2006, vol. 368, 1005-1011

- **ZENG LY et al.** A prompt method to quantitative assay of alpha-Gal on pig cell surface without injury. *Sichuan Da XUe Xue Bao Yi Xue Ban,* 2005, vol. 36 (3), 419-421

- **GALILI U et al.** *A sensitive assay for measuring alpha Gal epitope expression on cells by a monoclonal anti-Gal antibody,* 2005, vol. 1998 (65), 1129-1132

- **CHEN RH ; KADNER A ; MITCHELL RN ; SANTERRE DH ; ADAMS DH.** alpha-Gal and beta-Gal are preferentially expressed on porcine cardiac microvascular endothelium. *Transplant Proc,* 2000, vol. 32, 877-878

- **FENG W et al.** Distribution of the Alpha-Gal epitope on adult porcine bone tissue. *Transplant. Proceed.,* 2006, vol. 38, 2247-2251

- **GALILI U.** The alpha Gal epitope and the anti-Gal antibody in xenotransplantation and in cancer immunotherapy. *Immun. Cell Biol.,* 2005, vol. 83, 674-686

- **KONAKCI KZ ; BOHLE B ; BLUMER R ; HOETZENECKER W ; ROTH G ; MOSER B ; BOLTZ-NITULESCU G ; GORLITZER M ; KLEPETKO W ; WOLNER E.** Alpha-Gal on bioprostheses: xenograft immune response in cardiac surgery. *Eur J Clin Invest,* January 2005, vol. 35 (1), 17-23

- **PARK CS ; PARK SS ; CHOI SY ; YOON SH ; KIM WH ; KIM YJ.** Anti alpha-gal immune response following porcine bioprosthesis implantation in children. *J Heart Valve Dis.,* January 2010, vol. 19 (1), 124-130

- **LILA N ; MCGREGOR CG ; CARPENTIER S ; RANCIC J ; BYRNE GW ; CARPENTIER A.** Gal knock-out pig pericardium: new source of material for heart valve bioprostheses. *J Heart Lung Transplant.,* 29 December 2009, vol. 29 (5), 538-543

- **NASO F ; GANDAGLIA A ; LOP L ; SPINA M ; GEROSA G.** First quantitative assay of alpha-Gal in soft tissues: presence and distribution of the epitope before and after cell removal from xenogeneic heart valves. *Acta Biomater.,* 29 November 2010, vol. 7 (4), 1728-1734

- **MC GREGOR CGA ; CARPENTIER A ; LILA N ; LOGAN JS ; BYRNE GW.** Cardiac xenotransplantation technology provides materials for improbe bioprosthetic heart valves. *J. Thos. Card. Surg.,* 2011, vol. 141 (1), 269-275

- **SIMIONESCU DT.** Artificial Heart Valves, Wiley Encyclopedia of Biomedical Engineering. John Wiley & Sons Inc, 2006, 1-10

- **STACCHINO C ; BONA G ; BONETTI F ; RINALDI S ; DELLA CIANA L ; GRIGNANI A.** Detoxification process for glutaraldehyde-treated bovine pericardium: biological, chimica and mechanical characterization. *J Heart Vale Dis.,* March 1998, vol. 7 (2), 190-194